# EUROPEAN PATENT APPLICATION

(11) **EP 2 545 930 A1**
(43) Date of publication of application: **16.01.2013**
(21) Application number: 11753441.2
(22) Date of filing: 10.03.2011
(51) Int. Cl.: A61K 35/74, A23L 1/28, A61P 1/12, A61P 3/06, A61P 37/02, A61P 37/08

(54) **AGENT FOR CONTROLLING THE INCREASE AND DECREASE OF LACTOBACILLUS BIFIDUS IN COLON**

(30) Priority: 12.03.2010 JP 2010055816
(71) Applicant: Calpis Co., Ltd., Tokyo 150-0022 (JP)
(72) Inventor: HATANAKA Misaki, Sagamihara-shi Kanagawa 252-0206 (JP); NAKAMURA Yasunori, Sagamihara-shi Kanagawa 252-0206 (JP)
(74) Representative: Denison, Christopher Marcus
(86) International application number: PCT/JP2011/055661
(87) International publication number: WO 2011/111783

(57) **Abstract**

The present invention provides a means and a method for effectively increasing Bifidobacteria in the large intestine. Specifically, the present invention relates to an agent for increasing Bifidobacteria and/or reducing the decrease of Bifidobacteria in the large intestine, which comprises spores of *Bacillus subtilis* and a means of delivering the spores to the large intestine of a subject while maintaining the spore form *of Bacillus subtilis.*

## Description

### BACKGROUND OF THE INVENTION

### Field of the Invention

The present invention relates to an agent and a method for increasing Bifidobacteria and/or reducing the decrease of Bifidobacteria in the large intestine. More specifically, the present invention relates to the use of spores of *Bacillus subtilis* for increasing Bifidobacteria and/or reducing the decrease of Bifidobacteria in the large intestine.

### Background Art

To date, the following four strains have been reported as dominant Bifidobacterium strains in the adult intestine: *Bifidobacterium adolescentis, Bifidobacterium catenulatum, Bifidobacterium pseudocatenulatum,* and *Bifidobacterium longum* (Matsuki, T. et al., Appl. Environ. Microbiol. 70(1): 167-173, 2004). Bifidobacteria have been confirmed to be effective not only for intestinal regulation but also for immunoregulation, the improvement of blood lipid levels, and so on. Therefore, attempts have been made to increase Bifidobacteria in the intestines. Methods comprising orally ingesting Bifidobacteria resistant to gastric acid as probiotics and methods comprising ingesting oligosaccharides as prebiotics have been known as methods for increasing Bifidobacteria in the intestines.

Recently, it has become common to ingest lactic acid bacteria and the above Bifidobacteria as probiotics (JP Patent Publication No. 2007-091704 A). However, probiotics does not always result in similar outcomes in all humans because there are great differences between individuals and the colonization rates of ingested strains vary among individuals (Kagaku to Seibutsu 47(2): 78-80, 2009).

It has also become common to ingest growth factors for Bifidobacterium (e.g., oligosaccharides) as prebiotics. Such prebiotics have been found to be effective for the growth of *Bifidobacterium adolescentis*; however, they have not been confirmed to be effective for the growth of *Bifidobacterium longum* in the intestines (Asano et al., Nippon Nogeikagaku Kaishi, 75(10): 1077-1083, 2001).

Further, a composition for administering a Bifidobacterium formulation in combination with a growth accelerator for Bifidobacterium has been reported as a combined preparation of a probiotic and a prebiotic (JP Patent Publication No. 2009-296910 A).

There are reports on the usefulness of *Bacillus subtilis* for promotion of the growth of intestinal bacteria such as Bifidobacteria (JP Patent Publication No. 2009-296910 A; and Ushijima, Medicine and Biology, 128(1):9-14, 1994). According to the reports, germinated cells of *Bacillus subtilis* are effective for the growth of Bifidobacteria (Ushijima, 1994 *supra*), and spores of *Bacillus subtilis* are germinated in the small intestine such that germinated cells accelerate the growth of intestinal bacteria (JP Patent Publication No. 7-170975 (1995) A).

The present applicant also previously reported that spores *of Bacillus subtilis* had been confirmed to have intestinal regulatory action by administering the spores directly via the oral route to livestock animals (e.g., pigs and chickens) (see JP Patent No. 2528055 B).

### SUMMARY OF INVENTION

As described above, there are still demands in the art for a method and a means of increasing Bifidobacteria, which constructed the microbiota, and particularly of increasing *Bifidobacterium longum,* which is a major strain in microbiota.

An object of the present invention is to provide a means and a method for effectively increasing Bifidobacteria in the large intestine.

As a result of intensive studies in order to achieve the above object, the present inventors confirmed that some of spores of *Bacillus subtilis* germinate when passing through the digestive tract. The present inventors also have found that spores of *Bacillus subtilis* have effects of promoting the growth of Bifidobacteria in the large intestine, and that spores of *Bacillus subtilis* obviously have more significant effects of increasing *Bifidobacterium longum* and reducing the decrease of *Bifidobacterium longum,* in particular, than *Bacillus subtilis,* some of which are germinated. The present invention has been completed based on these findings.

Specifically, the present invention is described below.

[1] An agent for increasing Bifidobacteria and/or reducing the decrease of Bifidobacteria in the large intestine, which comprises spores of *Bacillus subtilis* and a means of delivering the spores to the large intestine of a subject while maintaining the spore form of *Bacillus subtilis.*

[2] The agent according to [1], which is substantially free of germinated cells of *Bacillus subtilis.*

[3] The agent according to [1] or [2], wherein *Bacillus subtilis* is *Bacillus subtilis* C-3102 (FERM BP-1096) or a mutant or derivative thereof.

[4] The agent according to any one of [1] to [3], wherein Bifidobacteria are *Bifidobacterium longum.*

[5] The agent according to any one of [1] to [4], which is used for oral administration and/or enteral administration.

[6] The agent according to any one of [1] to [5], which is used for food or drink products, feeds, or medicines.

[7] The agent according to any one of [1] to [6], which is used as an intestinal regulatory agent, anti-allergic agent, or agent for improving blood lipid levels.

[8] A method for producing a functional food or drink product, which comprises:
preparing the agent according to any one of [1] to [7], and
incorporating the agent into a food or drink product.

[9] A functional food or drink product containing the agent according to any one of [1] to [7] incorporated therein.

[10] A method for increasing Bifidobacteria and/or reducing the decrease of Bifidobacteria in the large intestine of a subject, which comprises:
delivering an effective amount of spores *of Bacillus subtilis* to the large intestine of the subject while maintaining the spore form of *Bacillus subtilis,* or
directly administering the spores to the large intestine of the subject.

[11] The method according to [10], wherein the subject is a human or non-human animal.

[12] The method according to [10] or [11], wherein *Bacillus subtilis* is *Bacillus subtilis* C-3102 (FERM BP-1096) or a mutant or derivative thereof.

[13] The method according to any one of [10] to [12], wherein Bifidobacteria are *Bifidobacterium longum.*

[14] The method according to any one of [10] to [13], wherein spores are administered to the subject via oral administration and/or enteral administration.

[15] An agent for use in increasing Bifidobacteria and/or reducing the decrease of Bifidobacteria in the large intestine, which comprises spores of *Bacillus subtilis* and a means of delivering the spores to the large intestine of a subject while maintaining the spore form of *Bacillus subtilis.*

The present invention provides an agent having excellent effects of increasing Bifidobacteria and/or reducing the decrease of Bifidobacteria. The agent of the present invention increases in and/or reduces the decrease of Bifidobacteria in the large intestine so as to contribute to the promotion of the health of a host and the prevention of a variety of diseases. Further, the agent of the present invention remains capable of increasing Bifidobacteria and/or reducing the decrease of Bifidobacteria even after high-temperature treatment. Therefore, the agent of the present invention can be preferably used in functional food or drink products and feeds.

### BRIEF DESCRIPTION OF DRAWINGS

Fig. 1 is a graph showing the increases of Bifidobacterium strains determined by microarray analysis after addition of spores and germinated cells of *Bacillus subtilis* to a large intestine model.

Fig. 2 is a graph showing the increases of Bifidobacterium strains determined by microarray analysis after addition of spores of *Bacillus subtilis* to a large intestine model.

Fig. 3 is a graph showing the decreases of *Bifidobacterium longum* determined by real-time PCR after addition of spores of *Bacillus subtilis* to a large intestine model.

### DETAILED DESCRIPTION OF THE INVENTION

The present invention is described in detail below. The present application claims a priority to Japanese Patent Application No. 2010-055816 filed on March 12, 2010, part or all of the contents described in the specification and/or drawings of which are incorporated herein by reference.

The present invention relates to the use of spores of *Bacillus subtilis* for increasing Bifidobacteria and/or reducing the decrease of Bifidobacteria in the large intestine. As shown in Example 2 below, it was confirmed that when spores of *Bacillus subtilis* are directly administered via the oral route, some of the cells germinate when passing through the gastrointestinal tract. Also, as shown in Example 3 below, spores of *Bacillus subtilis* were found to have effects of remarkably increasing *Bifidobacterium longum,* unlike the mixture of spores and germinated cells of *Bacillus subtilis.* Thus, the present invention is intended to deliver spores of *Bacillus subtilis* to the large intestine of a subject while maintaining the spore form of *Bacillus subtilis* so as to increase and/or reduce the decrease of Bifidobacteria, and especially *Bifidobacterium longum,* in the large intestine.

The present invention provides an agent for increasing Bifidobacteria and/or reducing the decrease of Bifidobacteria in the large intestine, which comprises spores of *Bacillus subtilis* and a means of delivering the spores to the large intestine of a subject while maintaining the spore form of *Bacillus subtilis.* The present invention also provides a method for increasing Bifidobacteria and/or reducing the decrease of Bifidobacteria in the large intestine of a subject, which comprises delivering an effective amount of spores of *Bacillus subtilis* to the large intestine of a subject while maintaining the spore form of *Bacillus subtilis* or directly administering the spores to the large intestine of a subject.

Any conventional *Bacillus subtilis* strain known in the art can be used in the present invention as long as the spore form of *Bacillus subtilis* is maintained and spores of *Bacillus subtilis* have desired effects of increasing Bifidobacteria and/or reducing the decrease of Bifidobacteria. In addition, such strain is preferably a strain that has been confirmed to be safe for animals in view of administration to/intake by animals. Specific examples of such *Bacillus subtilis* strain include *Bacillus subtilis* C-3102 (FERM BP-1096), *Bacillus subtilis* BN strain as well as the strains stocked at the RIKEN Bioresource Center (JCM2499, JCM11003, JCM20014, JCM20035, JCM20036, JCM20038, JCM20057, JCM20073, JCM20083, JCM20085, JCM20086, JCM20094, JCM20095, JCM20096, JCM20105, JCM20108, JCM20118, JCM20127, JCM20132, JCM20333, JCM20336, JCM20352, JCM20353, JCM20354, JCM20520, and JCM21228).

According to the present invention, the expression "effects of increasing Bifidobacteria and/or reducing the decrease of Bifidobacteria" means the capacity to increase the number of cells of a Bifidobacterium strain residing in the large intestine and/or reduce a decrease in the number of cells of a Bifidobacterium strain residing in the large intestine. The Bifidobacterium strains to be target for promotion of increase or reduction of decrease are not particularly limited as long as they belong to the genus *Bifidobacterium.* Examples thereof include *B. longum, B. adolescentis, B. catenulatum, B. angulatum, B. bifidum, B. breve, B. dentium, B. gallicum, B. globosum, B. infantis, B. subtile, B. asteroides, B. boum, B. choerinum, B. coryneforme, B. cuniculi, B. gallinarum, B. indicum, B. magnum, B. merycicum, B. minimum, B. psychraerophilum, B. pullorum, B. ruminantium, B. scardovii, B. thermacidophilum* subsp. *porcinum,* and *B. thermophilum.* In particular, according to the present invention, the above effects are effects of increasing and/or reducing the decrease of *Bifidobacterium longum,* which is a major bacterium in the intestinal flora. Such effects can be confirmed by, for example, incubating spores of *Bacillus subtilis* with one or more types of Bifidobacterium strain under conditions similar to the conditions in the large intestine environment and evaluating an increase or decrease in the number of cells of the Bifidobacterium strain. A specific method for determining the effects of increasing and/or reducing the decrease of Bifidobacteria may encompass the method using a microarray or real-time PCR described in Example 3 below.

Any *Bacillus subtilis* strain can be used in the present invention as long as it has been evaluated as having effects of increasing and/or reducing the decrease of Bifidobacteria by the above method or the like. An example of *Bacillus subtilis* having such desired effects is *Bacillus subtilis* C-3102 (FERM BP-1096). This strain has been confirmed to remarkably increase and reduce the decrease of Bifidobacteria, and especially *Bifidobacterium longum,* in the Examples described below. The strain has been deposited under Budapest Treaty by the present applicant with the International Patent Organism Depositary, National Institute of Technology and Evaluation (NITE) (previously called the Fermentation Research Institute, the Agency of Industrial Science and Technology, the Ministry of International Trade and Industry) (Tsukuba Central 6, 1-1-1 Higashi, Tsukuba, Ibaraki, 305-8566, Japan) under accession no. FERM BP-1096 as of December 25, 1985). The strain is available at the center. *Bacillus subtilis* C-3102 has been reported that can be used in animals by making use of its microbiota improvement action, meat quality improvement action, and intestinal regulatory action, as well as other actions (e.g., JP Patent Publication No. 04-024022 (1992) B and Suzuki et al., Chounai saikingaku zassi, vol. 18, no. 2, pp. 93-99 (2004)). Thus, *Bacillus subtilis* C-3102 has been confirmed to be safe in animals, including humans.

In addition, a mutant or derivative of any of the specific strains described above can be used in the present invention as long as it has effects of increasing and/or reducing the decrease of Bifidobacteria.

Spores of *Bacillus subtilis* are used in the present invention. The term "spore" refers to a spore-forming bacteria. Spores have resistance to temperatures and chemical substances. Spores germinate under conditions appropriate for bacterial growth and grow into vegetative cells (germinated cells). Whether or not a given *Bacillus subtilis* strain is a spore-form bacterium can be confirmed based on the heat resistance of the strain by examining the survival or nonsurvival of bacterial cells after high-temperature treatment. According to the present invention, *Bacillus subtilis* to be used contain spores of preferably 70% or more, more preferably 80% or more, and particularly preferably 90% or more of the total cells, and vegetative cells (germinated cells) of preferably less than 30%, more preferably less than 20%, and particularly preferably less than 10% of the total cells.

Spores of *Bacillus subtilis* can be prepared via culture under adequate conditions using a medium conventionally used for culture of microorganism. A natural medium, a synthetic medium, liquid medium or solid medium can be used as a culture medium as long as it contains a carbon source, a nitrogen source, a mineral salt, and other components and it enables culture of *Bacillus subtilis* with efficiency. Those skilled in the art can adequately select a known medium appropriate for a bacterial strain to be used. Examples of a carbon source that can be used include lactose, glucose, sucrose, fructose, galactose, and blackstrap molasses. Examples of a nitrogen source that can be used include organic nitrogen-containing substances such as peptone, casein hydrolysate, whey protein hydrolysate, and soy protein hydrolysate. Examples of a mineral salt that can be used include phosphate, sodium, potassium, and magnesium. Examples of an appropriate medium for culture of *Bacillus subtilis* include a TS (Trypticase Soy) agar medium and an HI (Heart Infusion) agar medium.

*Bacillus subtilis* can be cultured at 20°C to 50°C, preferably 30°C to 45°C, and particularly preferably approximately 37°C under aerobic conditions. Temperature conditions can be adjusted using a thermostatic bath, a mantle heater, a jacket, or the like. The format of culture includes static culture, shake culture, and tank culture. In addition, the period of culture can be determined to be 12 hours to 7 days and preferably 2 days to 3 days. It is preferable to maintain the pH of the medium at 5 to 9 and preferably 6 to 8 in the beginning of culture.

After culture, the obtained culture product of *Bacillus subtilis* can be directly used, or it may be further subjected to sterilization and crude purification via centrifugation, etc. and/or solid-liquid separation via filtration, etc. according to need. In addition, *Bacillus subtilis* used in the agent of the present invention may be preferably in the form of viable bacterial cells including wet bacterial cells and dried bacterial cells; however, it may be in the form of dead bacterial cells.

*Bacillus subtilis* may be further subjected to treatment to obtain a treated product of *Bacillus subtilis* according to need. Examples of such treatment are described below.

*Bacillus subtilis* can be prepared in the form of suspension or diluted solution by suspension or dilution in an adequate solvent. Examples of a solvent that can be used include water, physiological saline, and phosphate buffer saline (PBS).

A heated product can be prepared by heat treatment of *Bacillus subtilis.* In order to prepare such heated product, high temperature treatment (for example, at 60 °C to 100 °C) of *Bacillus subtilis* is performed for a certain period, for example, for approximately 10 minutes to 1 hour (e.g., approximately 10 to 30 minutes). Since spores *of Bacillus subtilis* are used in the present invention, the spores are viable even after high-temperature treatment and retain the desired effects.

A sterilized product can be prepared by sterilization treatment of *Bacillus subtilis.* In order to subject *Bacillus subtilis* to sterilization treatment, for example, a known technique of sterilization treatment such as filtration sterilization, radiation disinfection, superheat disinfection, or pressure disinfection can be used.

*Bacillus subtilis* can be processed into the form of a powdery product or granular product via drying. Drying methods include, but not particularly limited to, spray drying, drum drying, vacuum drying, and lyophilization, which can be used alone or in combination. Upon drying, conventionally used excipients may be added according to need.

The above examples of treatment may be used alone or in combinations where appropriate. According to the present invention, such treated *Bacillus subtilis* can be used for increasing and/or reducing the decrease of Bifidobacteria in the large intestine.

When an agent for increasing and/or reducing the decrease of Bifidobacteria in the large intestine which comprises *Bacillus subtilis* obtained above alone or in combination with other ingredients is mixed with a food or drink product, feed, or medicine for intake, the increase of and/or reduction of the decrease of Bifidobacteria in the large intestine and accompanying intestinal regulatory action, anti-allergic action, or blood lipid improvement action can be expected.

The agent for increasing and/or reducing the decrease of Bifidobacteria of the present invention (hereinafter sometimes referred to as "the agent of the present invention") comprises, as an active ingredient, *Bacillus subtilis* described above. It may comprise a single *Bacillus subtilis* strain or a plurality of different *Bacillus subtilis* strains. Further, it may comprise a combination of *Bacillus subtilis* strains that have been treated in different ways.

According to the present invention, it is necessary to deliver spores of *Bacillus subtilis* to the large intestine of a subject while maintaining the spore form of *Bacillus subtilis.* For such reason, spores of *Bacillus subtilis* are used for administration or intake in combination with a means of delivering spores to the large intestine of a subject while maintaining the spore form of *Bacillus subtilis* or are directly administered to the large intestine.

The expression "a means of delivering to the large intestine" used herein refers to, for example, a means that allows spores of *Bacillus subtilis* to be delivered to the large intestine (including the colon and the rectum) while preventing the spores from germinating in the stomach and the small intestine. An example of such means is a means that allows spores of *Bacillus subtilis* to pass through the stomach and the small intestine while maintaining the spore form of *Bacillus subtilis* by preventing the spores from germinating in a high-nutrient environment and/or a low-pH environment in the stomach and the small intestine. Such delivery means are well-known in the art and are not particularly limited. Examples thereof include enteric coatings, enteric capsules, enteric tablets, and liposomes. An enteric coating is formed with a composition or a combination of compositions for coating a formulation in a manner such that a formulation does not become dissolved or disintegrated in the stomach and the small intestine and is not modified in terms of structural characteristics. Examples of such enteric coating include polylactic acid, a lactic acid-glycolic acid copolymer, a cellulose ester derivative (e.g., cellulose acetate phthalate or carboxymethylcellulose), cellulose ether, alginate, a methyl acrylate copolymer, and Eudragit^{®} L/S. Enteric tablets can be obtained by coating tablets with an enteric coating. In addition, enteric capsules can be obtained by coating capsules with an enteric coating or by preparing capsules with materials used for an enteric coating and encapsulating spores of *Bacillus subtilis* and an appropriate carrier within the capsules. Such delivery means are described in, for example, WO2005/117921 A, WO2002/091833 A, WO2004/014403 A, JP Patent Publication No. 11-199494 (1999) A, and WO2008/114889 A. A person skilled in the art can readily understand the type of delivery means, a method for preparing a delivery means, and the use of a delivery means for spores of *Bacillus subtilis.*

A means of delivering spores of *Bacillus subtilis* to the large intestine can be adequately selected depending on the type or age of the subject of administration or intake of the spores and the dosage form and designed for a favorable mode of delivery.

In addition, methods for directly administering spores of *Bacillus subtilis* to the large intestine are known in the art. For example, enteral administration, intrarectal injection, or the like can be employed.

Further, in addition to spores of *Bacillus subtilis* used as active ingredients and a means of delivering them, additives described below, an agent for accelerating the growth of *Bacillus subtilis* or Bifidobacteria, a known intestinal regulatory agent, and other ingredients can be added alone or in combination thereof to the agent of the present invention if the desired effects are not inhibited.

The dosage form of the agent of the present invention includes, but not particularly limited to, oral formulations such as tablets, capsules, granules, powders, dust formulations, syrups, dry syrups, solutions, suspensions, and inhalers; enteral formulations; and injectable agents. Of these, the agent of the present invention is preferably in the form of an oral formulation. In addition, a liquid formulation such as a solution or suspension may be in the form such that a dosage form can be dissolved or suspended in water or a different adequate medium immediately before use. When the agent of the present invention is formed into tablets or granules, coating may be performed by a known method. Further, the agent of the present invention may be prepared as a controlled-release formulation such as a sustained-release formulation, a delayed-release formulation, or an immediate release formulation with the use of a technique known in the art.

The agent in the above dosage form can be prepared according to a conventional method by formulating conventionally used additives such as excipients, disintegrators, binders, wetting agents, stabilizers, buffering agents, lubricants, preservatives, surfactants, sweeteners, flavoring agents, aromatics, acidulants, and coloring agents into the ingredients described above in accordance with the dosage form. For example, in a case in which the agent of the present invention is prepared as a pharmaceutical composition, a pharmaceutically acceptable carrier or an additive can be incorporated into the agent of the present invention. Examples of such pharmaceutically acceptable carriers and additives include water, pharmaceutically acceptable organic solvents, collagen, polyvinyl alcohol, polyvinyl pyrrolidone, carboxyvinyl polymers, sodium alginate, water-soluble dextran, water-soluble dextrin, carboxymethyl starch sodium, pectin, xanthan gum, arabic gum, casein, gelatin, agar, glycerin, propylene glycol, polyethylene glycol, vaseline, paraffin, stearyl alcohol, stearic acid, human serum albumin, mannitol, sorbitol, lactose, surfactants acceptable as pharmaceutical additives, and artificial cell constructs such as liposome.

When the agent of the present invention contains the above additives and other agents such as an agent for accelerating the growth of *Bacillus subtilis* or Bifidobacteria and other intestinal regulatory agent, the content of spores of *Bacillus subtilis* used as an active ingredient may depend on the dosage form thereof. For example, the content is generally 0.01 % to 99% by mass, preferably 0.1% to 80% by mass, and more preferably 0.1% to 75% by mass. In order to achieve intake of the desirable amount of an active ingredient, it is desirable to prepare the agent of the present invention in a dosage form that allows management of the daily dose. In addition, the number of spores of *Bacillus subtilis* contained in the agent of the present invention is, for example, approximately 10⁴ cells/g to 10¹¹ cells/g.

Further, the agent of the present invention may further contain a variety of additives used for production of medicines, food or drink products, feeds, and other various substances. Examples of such substances and additives include a variety of fats and oils (e.g., plant oils such as soybean oil, corn oil, safflower oil, and olive oil, and animal fat and oil such as beef fat or sardine oil), herbal medicines (e.g., royal jelly and ginseng), amino acids (e.g., glutamine, cysteine, leucine, and arginine), polyalcohols (e.g., ethylene glycol, polyethylene glycol, propylene glycol, glycerin, and sugar alcohols such as sorbitol, erythritol, xylitol, maltitol, and mannitol), natural polymers (e.g., arabic gum, agar, water-soluble corn fibers, gelatin, xanthan gum, casein, gluten or gluten hydrolysate, lecithin, starch, and dextrin), vitamins (e.g., vitamin C and vitamin Bs), minerals (e.g., calcium, magnesium, zinc, and iron), dietary fibers (e.g., mannan, pectin, and hemicellulose), surfactants (e.g., glycerin esters of fatty acid and sorbitan esters of fatty acid), purified water, excipients (e.g., glucose, cornstarch, lactose, and dextrin), stabilizing agents, pH adjusting agents, antioxidants, sweeteners, flavoring agents, acidulants, coloring agents, and aromatics. For example, a substance effective for accelerating growth of *Bacillus subtilis* used as an active ingredient or Bifidobacteria to be increased can be selected. Examples of such growth accelerator include a growth medium for *Bacillus subtilis* or Bifidobacteria, an oligosaccharide, and a milk protein.

Further, in addition to the above active ingredient, a functional ingredient or an additive can be incorporated into the agent of the present invention. Examples thereof include taurine, glutathione, carnitine, creatine, coenzyme Q, glucuronic acid, glucuronolactone, Capsicum extract, ginger extract, cacao extract, guarana extract, garcinia extract, theanine, γ-aminobutyric acid, capsaicin, capsiate, a variety of organic acids, flavonoids, polyphenols, catechins, xanthine derivatives, indigestible oligosaccharides such as fructooligosaccharide, and polyvinyl pyrrolidone.

The amount of such additive can be adequately determined depending on the type of additive and the desirable amount. The content of the additive is generally 0.01 % to 90% by mass and preferably 0.1 % to 50% by mass of the total mass of the agent of the present invention.

Subjects of administration or intake of the agent of the present invention may be vertebrate animals. Specific examples thereof include mammals such as humans, primates (e.g., monkeys and chimpanzees), livestock animals (e.g., cattle, horses, pigs, and sheep), pet animals (e.g., dogs and cats), and experimental animals (e.g., mice and rats). Further, such subjects can be reptiles and birds. A human who needs to have the increase in Bifidobacteria in the large intestine is particularly preferable.

The dose (effective dose) of administration or intake of the agent of the present invention may depend on the age and body weight of a subject, an administration/intake route, and the number of doses for administration/intake, and can be changed extensively at the discretion of those skilled in the art to achieve desired effects. For example, for oral administration or intake, the dose of administration/intake of spores of *Bacillus subtilis* contained in the agent of the present invention is generally approximately 10⁴ cells/day to 10¹¹ cells/day. The content of *Bacillus subtilis* is not particularly limited and can be adequately adjusted in accordance with the degree of ease of production, and the preferable daily dose, for example. The agent of the present invention is safe and thus it is also possible to further increase the dose of intake. The daily dose of intake may be a single dose, or it may be divided into several doses. In addition, the frequency of administration or intake is not particularly limited, and it can be adequately selected depending on various conditions such as an administration/intake route, the age and body weight of a subject, and desired effects (e.g., therapeutic or preventive effects).

The administration/intake route of the agent of the present invention is not particularly limited, and includes oral administration/intake, and parenteral administration (e.g., enteral or intrarectal administration). Particularly preferably, the agent of the present invention is orally administered or taken.

The agent of the present invention increases and/or reduces the decrease of intestinal bacteria useful for a host in the large intestine of the host, and it specifically increases Bifidobacteria and/or reduces the decrease of Bifidobacteria, and especially *Bifidobacterium longum,* so as to promote the health of the host and prevent a variety of diseases. In addition, the agent of the present invention is highly safe and thus intake thereof can be easily continued for long time. Thus, the agent of the present invention can also be used for foods or drink products and feeds.

The agent of the present invention may be used in combination with a further medicine or a furthre treatment or prevention method. A further medicine and the agent of the present invention may be formulated into a single formulation. Alternatively, they may be formulated into separate formulations so as to be administered simultaneously or at intervals.

Further, upon use of the agent of the present invention, spores of *Bacillus subtilis* can be used in combination with a conventional method unless it influences the effects of the present invention. In addition, it is expected that even more remarkable effects will be achieved by using the agent of the present invention in combination with a conventional method than those achieved by using the agent alone.

As described above, the agent of the present invention has effects of increasing Bifidobacteria and/or reducing the decrease of Bifidobacteria in the large intestine and comprises conventionally used *Bacillus subtilis.* It is highly safe and thus free from worry about side effects. The spores of *Bacillus subtilis* contained as an active ingredient in the agent have resistance to high temperatures and chemical substances, allowing them to be stable during various forms of physical or chemical treatment used in the process of producing food or drink products. In addition, they have excellent preservative properties. Further, when the agent of the present invention is added to a variety of food or drink products, it does not even spoil the original flavors of food or drink products. Therefore, the agent of the present invention can be added to a variety of food or drink products for continuous intake thereof.

The food or drink product of the present invention contains the agent of the present invention described above. Examples of the food or drink product containing the agent of the present invention include all food or drink products into which the agent of the present invention can be incorporated, for example, food or drink products such as health food or drink products, functional food or drink products, and food or drink products for specified health use having effects of increasing Bifidobacteria and/or reducing the decrease of Bifidobacteria for health promotion.

Functional food or drink products are particularly preferable as food or drink products containing the agent of the present invention. The "functional food or drink product" according to the present invention means a food or drink product having predetermined functionality for organisms and encompasses, for example, so-called general health food or drink products such as food or drink products with health claims including food for specified health use (including qualified FOSHU [food for specified health use]) and food or drink products with nutrient function claims, food or drink products for special dietary uses, nutritional supplements, health supplements, supplements (e.g., those having a variety of dosage forms such as tablets, coated tablets, sugar-coated tablets, capsules, and liquid agents), and beauty food or drink products (e.g., diet food or drink products). The functional food or drink products of the present invention also encompass health food or drink products to which Health claim based on the food standards of Codex (Joint FAO/WHO Food Standards Program) is applied.

Specific examples of food or drink products include health food or drink products and nutritional supplements in preparation forms such as liquid diets (e.g., tube enteral nutritional supplements), tablet candies, tablets, chewable tablets, dust formulations, powders, capsules, granules, and tonic drinks; tea beverages such as green tea, oolong tea, and black tea; drinks or beverages such as soft drinks, jelly beverages, isotonic beverages, milk beverages, carbonated beverages, vegetable beverages, juice beverages, fermented vegetable beverages, fermented juice beverages, fermented milk beverages (e.g., yogurt), lactic acid bacteria beverages, milk beverages (e.g., coffee milk and fruit milk), beverages containing drink powders, cocoa beverages, milk, and purified water; spreads such as butter, jam, dried seasoning products, and margarine; mayonnaise; shortening; custard; dressings; bread; boiled rice; noodles; pasta; miso soup; tofu; yogurt; soup or sauce; and sweets (e.g., biscuits and cookies, chocolate, candies, cake, ice cream, chewing gum, and tablets).

The food or drink product of the present invention can be produced according to a conventional method by adding other food materials used for production of the above food or drink products, various nutrients, various vitamins, minerals, dietary fibers, and various additives (e.g., taste components, sweeteners, acidulants such as organic acids, stabilizers, and flavors), in addition to the agent of the present invention.

For the food or drink product of the present invention, those skilled in the art can adequately determine the amount of the agent of the present invention or spores of *Bacillus subtilis* formulated in consideration of the form of the food or drink product and the taste or texture that are required. Usually, an appropriate amount of the agent of the present invention is generally 0.001 to 100% by mass, preferably 0.01 to 80% by mass, and more preferably 0.01 to 50% by mass in total of spores of *Bacillus subtilis* in the agent of the present invention to be added. The agent of the present invention is safe, and thus the amount thereof in a food or drink product can be further increased. In order to achieve intake of the desirable amount of the agent of the present invention, it is desirable to prepare the agent of the present invention in a dosage form that allows management of the daily amount. As described above, the food or drink product of the present invention can be consumed in a form that allows management of the desirable amount of the agent of the present invention. Accordingly, a method for increasing Bifidobacteria and/or reducing the decrease of Bifidobacteria in the large intestine using the food or drink product can be provided.

The agent of the present invention may be incorporated into a food or drink product by an appropriate method available by those skilled in the art. For example, the agent of the present invention can be prepared in a liquid, gel, solid, powder, or granule form and then incorporated into a food or drink product. Alternatively, the agent of the present invention may be mixed or dissolved directly into raw materials for a food or drink product. The agent of the present invention may be applied to, coated onto, infiltrated into, or sprayed onto a food or drink product. The agent of the present invention may be dispersed uniformly or distributed unevenly in a food or drink product. A capsule containing the agent of the present invention may be prepared. An edible film or food coating agent may be wrapped around the agent of the present invention. Alternatively, the agent of the present invention may be prepared into a form such as a tablet after the addition of an appropriate excipient and others. The food or drink product comprising the agent of the present invention may further be processed. Such a processed product is also encompassed within the scope of the present invention.

In the production of the food or drink product of the present invention, a variety of additives as routinely used in food or drink products may be employed. Examples of the additives include, but are not limited to, color formers (e.g., sodium nitrite), coloring agents (e.g., gardenia pigments and Red 102), flavors (e.g., orange flavors), sweeteners (e.g., stevia and aspartame), preservatives (e.g., sodium acetate and sorbic acid), emulsifiers (e.g., sodium chondroitin sulfate and propylene glycol esters of fatty acid), antioxidants (e.g., disodium EDTA and vitamin C), pH adjusters (e.g., citric acid), chemical seasonings (e.g., sodium inosinate), thickeners (e.g., xanthan gum), swelling agents (e.g., calcium carbonate), antifoaming agents (e.g., calcium phosphate), binding agents (e.g., sodium polyphosphate), nutrition-enriching agents (e.g., calcium-enriching agents and vitamin A), and excipients (e.g., water-soluble dextrin). Functional raw materials such as *Panax ginseng* extracts, *Acanthopanax senticosus Harms* extracts, eucalyptus extracts, or du zhong tea extracts may further be added.

Further, the agent of the present invention can be formulated not only into food or drink products for humans but also into feeds for animals such as livestock (e.g., pigs and chickens), racehorses, and pets. Feeds are substantially equivalent to food or drink products except that they are given to non-human subjects. Therefore, the above descriptions of food or drink products can be applied mutatis mutandis to feeds.

The present invention is described in detail with reference to the following examples, although the present invention is not limited thereto.

### [Example 1] Preparation of spores

*Bacillus subtilis* C-3102 (FERM BP-1096) was cultured in a solid medium. Briefly, *Bacillus subtilis* C-3102 was cultured using a TS agar medium prepared by mixing "Trypticase Soy Broth" (product name; from BBL) (30 g/L) with 2% agar at 37°C for 2 to 3 days to obtain spores.

### [Example 2] Determination of the germination percentage of Bacillus subtilis using an artificial human intestinal model

A reproduced model of the stomach and the small intestine (TIM-1 system), which is an artificial intestinal model (TNO intestinal model: TIM) developed by TNO (Netherlands), was used to determine the germination percentage of spores of *Bacillus subtilis.*

Briefly, samples of each test group were introduced into the TIM-1 developed as an artificial model of the stomach and the small intestine by TNO. Sample was passed through the system for 6 hours, and the effluent was collected every hour, followed by determination of the germination percentage for the effluent samples. The TIM-1 model used herein is described in detail in Havenaar, R. and Minekus, M. Dairy Industries International 61:17-23, 1996, and Marteau, P. et al. J Dairy Sci 80:1031-1037, 1996.

A homogenized standard European continental breakfast (protein content: 12%; fat content: 32%; and carbohydrate content: 56%) was used as a dietary component with the addition of spores of *Bacillus subtilis* C-3102 (1 x 10¹⁰ CFU in total) prepared as described in Example 1. Experimentation was carried out (n = 2).

Bacterial cell counts that had been heated at 65°C for 30 minutes making use of the heat-resistant properties of spores were designated as "spores," and unheated bacterial cells were designated as "spores + germinated cells (vegetative cells)." The germination percentage was calculated by the following formula for evaluation: the number of unheated bacterial cells / the number of added bacterial cells × 100 (%) - the number of heated bacterial cells / the number of added bacterial cells × 100 (%). The number of bacterial cells was determined by a plating method.

For samples obtained by sampling, the percentage of heated bacterial cells was compared with that of unheated bacterial cells. The cumulative survival rate (the number of bacterial cells at a given time point / the number of added bacterial cells × 100%) was calculated for comparison of the results obtained at a given time point. The results are shown in Table 1.

**Table 1**

| Sample | Spores + germinated cells (unheated) | Spores (heated) |
|---|---|---|
| Added sample (1 × 10¹⁰ spores) | 100% | 100% |
| Collected ileal content (sample collected after 6 hours) | 74% | 64% |
| Collected ileal content (sample collected after 6 hours + residue) | 99% | 85% |

Based on the results shown in Table 1, the following germination percentage (%) of bacterial cells was determined for a mixture of the sample collected after 6 hours and the digested residue: 99/100 × 100 - 85/100 × 100 = 14%. This revealed that approximately 14% of the total spores of *Bacillus subtilis* germinate after passing through the stomach and small intestine.

### [Example 3] Variations in the intestinal flora using the artificial human large intestine model

### (1) Detection of intestinal flora bacteria by microarray analysis

As in the case of Example 2, a TIM-1 (a reproduced model of the stomach and the small intestine) and a TIM-2 model (a reproduced large intestine model), which are artificial intestinal models (TNO intestinal models: TIM) developed by TNO (Netherlands), were used for evaluation of variations in the human microbiota. Briefly, a TIM-2 developed as an artificial large intestine model by TNO was used for evaluation of variations in the microbiota by a microarray method. The TIM-2 is described in detail in Venema, K. et al. Nutrition 24(12): 558-564, 2000. In addition, the microarray used herein was an IChip, which is an microarray for intestinal flora analysis developed by TNO and contains probes for 360 bacterial strains residing in the human intestine (Maathuis, A. et al. FASEB J. 22 (Meeting Abstract Supplement): 1089.7, 2008).

The following were designated as test groups: a group for which the sample collected in Example 2, which had passed through the TIM-1 model for 6 hours, was used (#1) and a control group for which the dietary component alone was used (#2); and a group for which spores of *Bacillus subtilis* (1 × 10¹⁰ CFU in total) prepared in Example 1 were used (#3) and a control group for which no spores of *Bacillus subtilis* were used (#4). That is, sample #1 contained the digest of the dietary component (nutrient), spores, and germinated cells, sample #2 contained the digest of the dietary component, sample #3 contained the medium and spores, and sample #4 contained the medium alone. The medium used herein contained the following components: pectin (0.6 g/day), xylan (0.6 g/day), arabinogalactan (0.6 g/day), amylopectin (0.6 g/day), amylopectin (0.6 g/day), casein (3.0 g/day), starch (5.0 g/day), Tween 80 (2.16 g/day), bactotryptone (3.0 g/day), and bile (0.05 g/day).

The human microbiota of a healthy subject (Dutch) was inoculated into TIM-2, followed by preculture for 16 hours for adaptation. The samples for the test groups (#1 and #2, or #3 and #4) were separately added to TIM-2 and allowed to act thereon for 72 hours for experimentation (n = 2 for each test group).

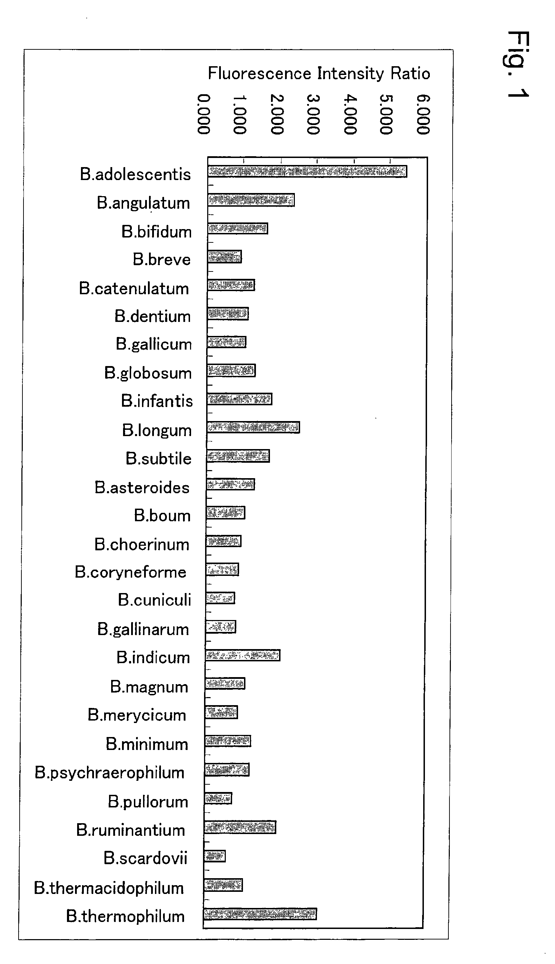

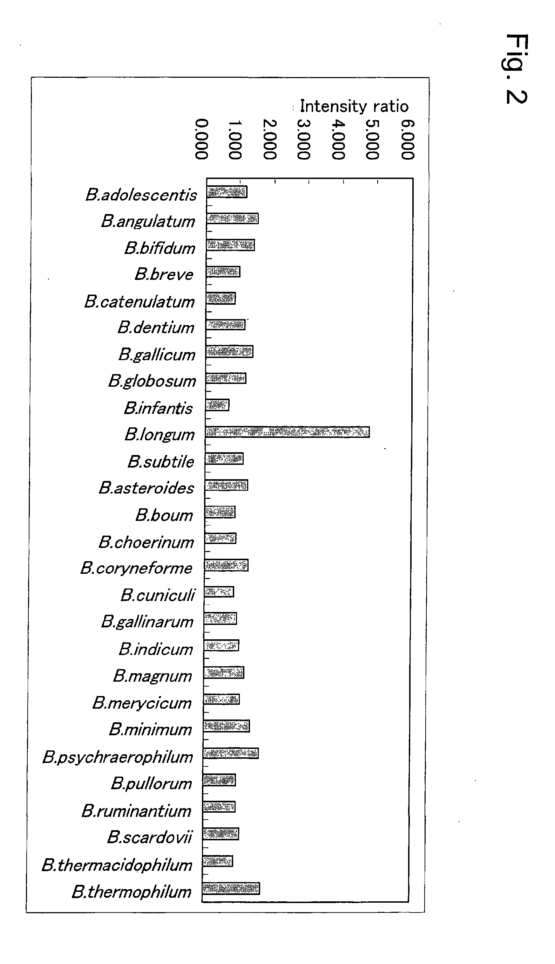

RNAs were extracted from the contents of the artificial intestine before and after experimentation and compared by a microarray method using an IChip. In particular, the number of cells of a Bifidobacterium strain constituting the microbiota before experimentation (0 hour) and that after experimentation (72 hours) was compared based on the increase or decrease in fluorescence intensity for each test group. The increase/decrease was compared between the control group (#2 or #4) and the *Bacillus subtilis* administration group (#1 or #3). In a case in which at least a two-fold increase or decrease in the fluorescence intensity ratio of the test group to the control group was confirmed for a Bifidobacterium strain, such strain was evaluated as having experienced an increase or decrease in the number of its cells. The results are shown in Fig. 1 (#1/#2) and Fig. 2 (#3/#4).

As shown in Figs. 1 and 2, the number of cells was confirmed to have increased for the following bacteria in the case of test group #1, the sample of which had contained both spores and germinated cells: *Bifidobacterium adolescentis, Bifidobacterium angulatum, Bifidobacterium longum, Bifidobacterium indicum,* and *Bifidobacterium thermophilum* (Fig. 1). To the contrary, in the case of test group #3, for which spores had been directly administered to the large intestine model, the number of cells increased only for *Bifidobacterium longum.* The increase confirmed for test group #3 was significantly greater than that confirmed for test group #1, the sample of which had contained both spores and germinated cells (Fig. 2).

The above results revealed that when spores of *Bacillus subtilis,* not germinated cells, are allowed to reach the large intestine, the spores are more effective for increasing a Bifidobacterium strain, and especially *Bifidobacterium longum,* than *Bacillus subtilis,* approximately 14% of which have germinated after passing through the gastrointestinal tract.

### (2) Detection of intestinal flora bacteria by real-time PCR

DNA was extracted by a phenol chloroform method from the contents of the artificial intestine for each test group obtained before and after experimentation in (1) above. In order to confirm the increase or decrease of *Bifidobacterium longum,* copy number of the 16S ribosomal DNA was determined by real-time PCR with reference to primer sequences specific to the 16S rDNA sequence of *Bifidobacterium longum* (see Malinen, E. et al. Microbiology 149:269-277, 2003).

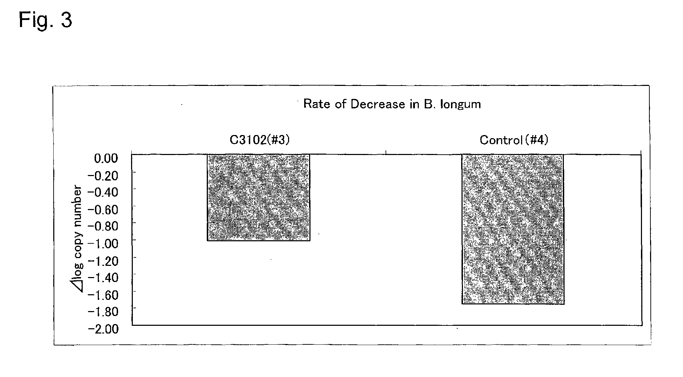

The results are shown in Table 2 and Fig. 3 (#3/#4).

**Table 2**

| | B. longum log (copy number) | |
|---|---|---|
| | C3102(#3) | Control (#4) |
| 0 hour | 6.01 | 5.80 |
| 72 hours | 5.00 | 4.05 |
| Rate of decrease | -1.01 | -1.75 |

The results shown in Table 2 and Fig. 3 indicate that spores of *Bacillus subtilis* reduced the decrease of the number of cells of *Bifidobacterium longum.* In the case of the control group, the decreased number of cells of *Bifidobacterium longum* might have resulted from evaluation carried out under experimental conditions in which medium components alone had been administered and thus the optimal nutrition status was not achieved. However, the results show that spores of *Bacillus subtilis* can remarkably reduce the decrease of the number of cells of *Bifidobacterium longum.* In addition, it is expected that the number of cells of *Bifidobacterium longum* can be increased under conditions that allow the number of the cells to be maintained.

The results obtained in (1) and (2) above revealed that spores of *Bacillus subtilis,* not germinated cells, can increase Bifidobacteria and reduce the decrease of Bifidobacteria, and especially *Bifidobacterium longum,* in the large intestine.

Further, it is expected that spores of *Bacillus subtilis* also can increase the number of cells of a different Bifidobacterium strain with changes to experimental conditions.

All publications, patents, and patent applications cited herein are incorporated herein by reference in their entirety.

### INDUSTRIAL APPLICABILITY

The present invention provides an agent having excellent effects of increasing Bifidobacteria and/or reducing the decrease of Bifidobacteria. The agent of the present invention can promote the health of a host by increasing and/or reducing the decrease of Bifidobacteria in the large intestine so as to contribute to prevention of various diseases. Further, even after high-temperature treatment, the agent remains capable of increasing and/or reducing the decrease of Bifidobacteria. Thus, the agent of the present invention can be preferably used in functional food or drink products and feeds.

## Claims

1. An agent for increasing Bifidobacteria and/or reducing the decrease of Bifidobacteria in the large intestine, which comprises spores of *Bacillus subtilis* and a means of delivering the spores to the large intestine of a subject while maintaining the spore form of *Bacillus subtilis.*

2. The agent according to claim 1, which is substantially free of germinated cells of *Bacillus subtilis.*

3. The agent according to claim 1 or 2, wherein *Bacillus subtilis* is *Bacillus subtilis* C-3102 (FERM BP-1096) or a mutant or derivative thereof.

4. The agent according to any one of claims 1 to 3, wherein Bifidobacteria are *Bifidobacterium longum.*

5. The agent according to any one of claims 1 to 4, which is used for oral administration and/or enteral administration.

6. The agent according to any one of claims 1 to 5, which is used for food or drink products, feeds, or medicines.

7. The agent according to any one of claims 1 to 6, which is used as an intestinal regulatory agent, anti-allergic agent, or agent for improving blood lipid levels.

8. A method for producing a functional food or drink product, which comprises:
preparing the agent according to any one of claims 1 to 7; and
incorporating the agent into a food or drink product.

9. A method for increasing Bifidobacteria and/or reducing the decrease of Bifidobacteria in the large intestine of a subject, which comprises:
delivering an effective amount of spores of *Bacillus subtilis* to the large intestine of the subject while maintaining the spore form *of Bacillus subtilis,* or
directly administering the spores to the large intestine of the subject.

10. The method according to claim 9, wherein *Bacillus subtilis* is *Bacillus subtilis* C-3102 (FERM BP-1096) or a mutant or derivative thereof.

11. The method according to claim 9 or 10, wherein Bifidobacteria are *Bifidobacterium longum.*
